# EUROPÄISCHE PATENTSCHRIFT

(11) **EP 3 986 659 B1**
(45) Veröffentlichungstag und Bekanntmachung des Hinweises auf die Patenterteilung: **30.09.2026**
(21) Anmeldenummer: 20726770.9
(22) Anmeldetag: 14.05.2020
(51) Int. Cl.: B23K 26/03, B23K 26/142, B23K 26/38, G01B 11/24, B23K 101/16, B23K 101/18

(54) **VERFAHREN ZUM BEWERTEN EINER LASERSCHNITTKANTE, MOBILES ENDGERÄT UND SYSTEM MIT SOLCHEM ENDGERÄT**
METHOD FOR EVALUATING A LASER CUTTING EDGE, MOBILE TERMINAL AND SYSTEM WITH SUCH TERMINAL
PROCÉDÉ D'ANALYSE D'UNE ARÊTE DE COUPE AU LASER, APPAREIL TERMINAL MOBILE ET SYSTÈME AVEC UN TEL TERMINAL

(30) Priorität: 24.06.2019 DE 102019209088
(43) Veröffentlichungstag der Anmeldung: 27.04.2022
(73) Patentinhaber: TRUMPF Werkzeugmaschinen SE + Co. KG, 71254 Ditzingen (DE)
(72) Erfinder: TATZEL, Leonie Felica, 70825 Korntal (DE); KIEFER, Manuel, 74889 Sinsheim (DE); OTTNAD, Jens, 76199 Karlsruhe (DE)
(74) Vertreter: Trumpf Patentabteilung
(86) Internationale Anmeldenummer: PCT/EP2020/063545
(87) Internationale Veröffentlichungsnummer: WO 2020/259920

(56) Entgegenhaltungen:
- EP-A1- 3 301 642
- CN-U- 204 997 230
- JP-A- H11 129 083
- US-A1- 2018 082 416
- US-A1- 2019 005 356

## Beschreibung

Die Erfindung betrifft ein Verfahren zum Bewerten einer Laserschnittkante, ein mobiles Endgerät sowie ein System mit einem solchen Endgerät, siehe Ansprüche 1, 7 und 11.

Laserschneiden, auch Laserstrahlschneiden, bezeichnet das Durchtrennen von Festkörpern mittels kontinuierlicher oder gepulster Laserstrahlung durch Materialablation. Auf diese Weise können Metalle, Kunststoffe, Glas und organische Materialien geschnitten werden. Dabei müssen die Parameter der Laserstrahlung, wie Wellenlänge, mittlere Leistung, Pulsenergie und Pulsdauer der Applikation entsprechend angepasst werden. Zusätzlich stellt eine häufig konzentrisch zum Laser angeordnete Schneiddüse ein Prozessgas bereit, das die Fokussieroptik vor Dämpfen und Spritzern schützt und weiterhin den abgetragenen Werkstoff aus der Schnittfuge treibt. Die Schnittkante kann insbesondere beim Schneiden von Metall, insbesondere Blech, sehr unterschiedlich ausfallen. Je nach der im Wirkbereich erreichten Temperatur und zugeführten Prozessgasart stellen sich unterschiedliche Aggregatzustände des Fugenwerkstoffs ein. Eine hohe Schnittqualität ist durch eine geringe Riefenbildung auf den Schnittkanten und die fehlende Gratbildung an der Unterseite des Schnittes charakterisiert. Durch den kontinuierlichen Vorschub und das resultierende Schmelzen des Materials kann der Werkstoff auf den Schnittkanten verfestigen. Die Erstarrung kann hierbei wellenförmig erfolgen, womit eine charakteristische Riefenstruktur bzw. die Rauheit des Schnittes einhergehen kann. Eine Bart- oder Gratbildung wird häufig einer zu geringen antreibenden Kraft der Gasströmung zugeschrieben. Schmelztropfen an der Schnittunterkante können erstarren und bilden einen mehr oder minder stark anhaftenden Bart/Grat. Zu den Parametern, die die Schnittqualität beeinflussen, gehören u. a. die Fokuslage, die Vorschubgeschwindigkeit, die Laserleistung, die Intensitätsverteilung oder auch der Schneidgasdruck. Die Bewertung einer Schnittkante erfolgt häufig durch Betrachten oder manuelles Fühlen des Arbeiters oder durch Messen eine Höhenprofils mit teurer Mess-Sensorik. Je nach nachfolgender Verwendung des geschnittenen Werkstücks werden sehr unterschiedliche, zum Teil auch sehr hohe Anforderungen an die Schnittkanten-Qualität gestellt.

Aus der JP H11 129083 A (offenbarend den Oberbegriff der Ansprüche 1, 7 und 11) ist ein Verfahren zum Bewerten einer Laserschnittkante mittels Bildverarbeitung bekannt.

In US 2006/0049158 A1 wird ein Verfahren zur Steuerung eines automatischen Laserprozesses angegeben, bei dem mit einer Kamera das Ergebnis des Prozesses aufgenommen wird und für die weitere Steuerung des Prozesses verwendet wird.

Demgegenüber ist es Aufgabe der Erfindung, die Qualität bei der Bewertung des Prozessergebnisses weiter zu verbessern.

Diese Aufgabe wird gelöst durch ein Verfahren gemäß Patentanspruch 1, ein mobiles Endgerät gemäß Patentanspruch 7 sowie ein System mit einem solchen Endgerät gemäß Patentanspruch 11.

Die abhängigen Ansprüche betreffen vorteilhafte Weiterbildungen der Erfindung.

Ein Verfahren zum Bewerten einer Laserschnittkante eines Werkstücks weist zumindest die folgenden Schritte auf:
A) Erfassen von Bilddaten der Laserschnittkante und ihrer Umgebung;
B) Segmentierung der Bilddaten und Identifikation eines interessierenden Segmentes der Bilddaten, wobei das interessierende Segment Bilddaten der Laserschnittkante aufweist;
C) Durchführung einer Bildqualitätserkennung für das interessierende Segment;
D) Generierung einer Ausgabe für einen Nutzer, basierend auf der Bildqualitätserkennung.

Das Werkstück besteht vorzugsweise aus festem Werkstoff, insbesondere aus stark reflektierendem Werkstoff, wie z.B. Glas oder Metall, insbesondere Blech.

Im vorliegenden Verfahren wird in Schritt A) mittels einer Kamera ein Bild der Laserschnittkante und ihrer Umgebung erfasst. In digitalisierter Form wird das erfasste Bild als Bilddaten in einer Bilddatei gespeichert. Ein Pixel ist dabei ein einzelner Bildpunkt eines Bildes. Es ist möglich, dass der von der Kamera erfasste Bildausschnitt Bereiche umfasst, die nicht die Laserschnittkante betreffen, sondern von der Laserschnittkante entfernt auf dem Werkstück oder sogar außerhalb des Werkstücks liegen. Diese sogenannte Umgebung der Laserschnittkante ist für die Beurteilung der Bildqualität weniger relevant als der Bildausschnitt, der direkt die Laserschnittkante betrifft.

Die Segmentierung der Bilddaten in Schritt B) erfolgt mittels elektronischer Bildverarbeitung und hat die Identifikation eines interessierenden Segments des Bildes zum Ergebnis, wobei das interessierende Segment des Bildes Bilddaten der Laserschnittkante aufweist. Für ein gutes Funktionieren des Verfahrens sollte das interessierende Segment ganz überwiegend Bilddaten der Laserschnittkante aufweisen und nur zu einem ganz geringen Teil Bilddaten der Umgebung der Laserschnittkante.

Entsprechend der Erfindung wird in Schritt B) zu jedem Pixel der Bilddaten ermittelt, ob das Pixel Teil der Laserschnittkante ist oder Teil der Umgebung der Laserschnittkante ist. Weiter bevorzugt wird als Ergebnis von Schritt B) zu jedem Pixel der Bilddaten abgespeichert, ob es Teil der Laserschnittkante ist oder Teil der Umgebung der Laserschnittkante ist.

In einer Ausführungsform der Erfindung wird Schritt B) mittels eines neuronalen Netzes ausgeführt. Das neuronale Netz, auch künstliches neuronales Netz genannt, ist ein üblicherweise auf einem Computer betriebener Algorithmus. Es weist eine Eingabeschicht und eine Ausgabeschicht und optional eine oder mehrere Zwischenschichten auf und kann, z. B. mittels "Deep Learning" trainiert werden. In der Eingabeschicht können dem neuronalen Netz Daten zugeführt werden, z.B. Bilddaten. In der Ausgabeschicht kann das neuronale Netz Vorschläge für ein Ergebnis ausgeben, z.B. Vorschläge für die Segmentierung der Bilddaten. Diese Vorschläge können vom neuronalen Netz zusätzlich jeweils mit einem Wert versehen sein, der die Wahrscheinlichkeit ausgibt, mit dem der Algorithmus die Segmentierung für erfolgreich durchgeführt bewertet. Die Korrektheit der Ausgabedaten kann dann von einem Nutzer bewertet werden. Diese Bewertung eines Nutzers kann das neuronale Netz verwenden, um seinen Algorithmus oder seine Algorithmen zu verbessern. Dazu können in der oder den Zwischenschicht(en) die Daten der Eingabeschicht mit Faktoren oder Rechenfunktionen mit anderen Daten verknüpft werden und so neue Daten in einer Zwischenschicht erzeugt werden. Die anderen Daten können Daten dieser oder einer weiteren Zwischenschicht oder der Ausgabeschicht sein. Dieses Anpassen der Faktoren oder Rechenfunktionen kann man als ,Training' des neuronalen Netzes bezeichnen. Durch das Training des neuronalen Netzes kann dieses aus vergangenen Erfahrungen lernen. In einer Trainingsphase des neuronalen Netzes können auch Beispiel-Bilder einer Trainingsdatenbank verwendet werden, in denen Laserschnittkanten vor einem (beispielsweise einfarbigen, insbesondere grünen) Hintergrund abgebildet sind. Für diese Beispielbilder kann mit Bildverarbeitung den Pixeln ein Label hinzugefügt werden, das angibt, welches Pixel zur Laserschnittkante gehört und welches zum (beispielsweise grünen) Hintergrund. Die Sammlung dieser Labels und der zugehörigen Pixel wird als "Ground Truth" des zugehörigen Beispielbildes bezeichnet. Des Weiteren kann der grüne Hintergrund durch andere Hintergründe ersetzt werden und so reale Aufnahmeszenarien simuliert werden. Dadurch kann die Trainingsdatenbank vergrößert werden.

In einer Ausführungsform der Erfindung wird zur Erkennung des interessierenden Segments eine Kombination eines "Spatial Pyramid Pooling" Algorithmus mit einem "Encoder Decoder Algorithmus" verwendet. Bevorzugt werden die beiden Algorithmen so kombiniert, dass vielfältige semantische Informationen in dem Encoder Modul enthalten sind und detaillierte Objektgrenzen, z. B. Grenzen der Laserschnittkante, von einem simplen und effektiven Decoder Modul erhalten werden. Das Encoder Modul erlaubt es mittels eines "Atrous Convolution" Algorithmus, Merkmale in beliebiger Auflösung zu extrahieren. Ein Beispiel eines geeigneten Algorithmus ist das DeepLabV3+ Netz, wie es z. B. in Chen, L.-C., Zhu, Y., Papandreou, G., Schroff, F., Adam, H., "Encoder-Decoder with Atrous Separable Convolution for Semantic Image Segmentation" (arXiv:1802.02611v3 [cs.CV] 22 Aug 2018) beschrieben wird.

In Schritt C) wird eine Bildqualitätserkennung des interessierenden Segmentes durchgeführt. Die Bildqualitätserkennung wird dabei auf das interessierende Segment beschränkt, da dieses in erster Linie die Laserschnittkante darstellt. Die Bildqualitätserkennung für die Umgebung der Laserschnittkante, also die Bereiche des Bildes, die nicht die Laserschnittkante darstellen, ist von geringerer Relevanz für die Bewertung der Qualität von Laserschnittkanten.

Schritt C) umfasst bevorzugt einen oder mehrere Aspekte von Bildqualität, z. B. eine Belichtungserkennung und/oder eine Schärfeerkennung.

Für die Schärfeerkennung werden bevorzugt die Bilddaten des Bildes in den Frequenzbereich transformiert. Die Transformation in den Frequenzbereich kann z. B. mittels einer Fourier-Transformation erfolgen. Die Schärfeerkennung erfolgt dann bevorzugt anhand der Bilddaten im Frequenzbereich. In einer Ausführungsform der Erfindung wird für die Schärfeerkennung im Frequenzbereich ein Algorithmus verwendet, der allein mit den aufgenommenen Bilddaten im interessierenden Segment arbeitet, also kein Vergleichsbild mit guter oder zufriedenstellender Schärfe benötigt. Ein Beispiel eines geeigneten Algorithmus ist eine NR-IQA Methode, wie sie z. B. in De, K., Masilamani, V., "Image Sharpness Measure for Blurred Images in Frequency Domain" (Procedia Engineering 64 (2013) 149-158) beschrieben wird.

Das Verfahren kann es einem Nutzer ermöglichen, ein Bild der Laserschnittkante aufzunehmen, das von der Qualität ausreichend ist, die Qualität der Schnittkante selbst zu ermitteln. In Schritt D) erhält der Nutzer dabei beispielsweise Rückmeldung in Bezug auf die Einstellung bestmöglicher Parameter für die Kamera und/oder die Optimierung von z. B.:
Schärfe in den 3 Dimensionen, stationäres bestmögliches Kontrastverhältnis, Bewegungsunschärfe;
Beleuchtungs-Intensität: Vermeidung von Über und Unterbelichtung sowie Anwendung von HDR-Verfahren (High Dynamic Range) für eine Aufweitung der maximalen Dynamik;
Rauschen: Optimierung ISO-Parametrierung;
Gamma-Korrektur: Automatische Parameter Anpassung an Umgebungslicht, z. B. Tageslicht, Neon-Lampe, etc., für realitätsnahe Farbwiedergabe;
Blende: Anpassung an höchst mögliche Tiefenschärfe

Die Ermittlung der Qualität der Schnittkante selbst kann dann in einem Schritt E) des Verfahrens beispielsweise durch das Betrachten des Bildes der Laserschnittkante und/oder maschinell durch Bildverarbeitung erfolgen.

In einer Ausführungsform des Verfahrens wird vor Schritt A) die Laserschnittkante mittels eines Laserstrahls und insbesondere unter Einsatzes eines Druckluftstrahls geschnitten. Dieses Laserschneiden wird vorzugsweise mittels einer Laserschneidmaschine durchgeführt. Bevorzugt wird das Ergebnis der Bewertung der Qualität der Laserschnittkante an die Laserschneidmaschine zurückgekoppelt und kann somit künftige Schneidvorgänge beeinflussen.

In einer Ausführungsform des Verfahrens werden vor Schritt A) Parameter betreffend die Laserschnittkante und/oder das Werkstück empfangen. Der Empfang kann beispielsweise durch eine Eingabe durch den Nutzer erfolgen. Der Empfang kann auch durch den Empfang von Daten von der Laserschneidmaschine oder von einer übergeordneten Steuerung erfolgen. Zusätzlich kann z. B eine Kennung des Werkstücks oder die Form des Werkstücks von der Kamera erfasst werden und dem Nutzer ein Vorschlag für Parameter angezeigt werden.

Die Aufnahme der Bilddaten erfolgt mittels einer Kamera. Die Kamera ist ein Bildaufnahmegerät, insbesondere im sichtbaren Bereich. Eine Kamera kann auch ein System aus mehreren Aufnahmevorrichtungen und einer Bildverarbeitungskomponente sein, die aus den mehreren Bildern ein Gesamtbild erzeugt. Bevorzugt ist die Kamera eingerichtet, Bilddaten in digitaler Form auszugeben.

Ein mobiles Endgerät mit einer Kamera, die eingerichtet ist, Bilddaten einer Laserschnittkante mit Umgebung zu erfassen, kann bei der Ausführung des Verfahrens eingesetzt werden.

Das mobile Endgerät ist eingerichtet, die Bilddaten an eine Recheneinheit zu übermitteln und von der Recheneinheit Bilddaten zu empfangen, wobei die empfangenen Bilddaten segmentiert sind und in den empfangenen Bilddaten ein interessierendes Segment identifiziert ist, wobei das interessierende Segment Bilddaten der Laserschnittkante aufweist; und wobei die empfangenen Bilddaten das Ergebnis einer Bildqualitätserkennung für das interessierende Segment umfassen. Das mobile Endgerät ist somit eingerichtet, Bilddaten zu empfangen, die gemäß der Schritte B) und C) des Verfahrens in einer Recheneinheit verarbeitet werden.

In der Erfindung ist die Recheneinheit, in der die Schritte B) und C) des Verfahrens ausgeführt werden, in dem mobilen Endgerät angeordnet.

Dies kann zum Beispiel der Fall sein, wenn das mobile Endgerät als Mobiltelefon oder Tablet-Computer ausgebildet ist.

Das mobile Endgerät weist entsprechend der Erfindung eine Ausgabeschnittstelle auf, die eingerichtet ist, eine Ausgabe für einen Nutzer zu generieren, wobei die Ausgabe auf dem Ergebnis der Bildqualitätserkennung basiert. Eine solche Ausgabeschnittstelle ist zum Beispiel im mobilen Endgerät vorhanden, wenn es als Mobiltelefon ausgebildet ist. Der Nutzer kann dann mit der Kamera des Mobiltelefons ein Bild der Laserschnittkante aufnehmen und bekommt über das selbe Mobiltelefon eine Rückmeldung, ob die Qualität des Bildes ausreichend ist und welche Parameter, z. B. Bildausschnitt, Beleuchtung etc. er noch ändern muss, um bei der nächsten Aufnahme eine bessere Bildqualität zu erzielen.

In einer Ausführungsform der Erfindung weist das mobile Endgerät mindestens eine Schnittstelle zum Empfang von Parametern betreffend die Laserschnittkante und/oder das Werkstück auf. Bevorzugt werden die Parameter vor Schritt A) des Verfahrens empfangen. Der Empfang kann beispielsweise durch eine Eingabe durch den Nutzer über eine Eingabeschnittstelle erfolgen. Der Empfang kann auch durch den Empfang von Daten von der Laserschneidmaschine oder von einer übergeordneten Steuerung, z. B. über eine drahtlose Kommunikationsschnittstelle, erfolgen.

Schritte des Verfahrens laufen entsprechend der Erfindung auf Elementen eines Systems ab, siehe Anspruch 11. Das System umfasst dabei eine Laserschneidmaschine und ein mobiles Endgerät umfassen. Die Laserschneidmaschine kann dabei eingerichtet sein, mittels eines Laserstrahls und insbesondere zusätzlich mittels eines Druckluftstrahls eine Laserschnittkante in ein Werkstück zu schneiden. Das mobile Endgerät ist wie ober beschrieben ausgebildet.

Das beschriebene Verfahren, das beschriebene mobile Endgerät und das beschriebene System können die Erzielung von höchstmöglicher Bildqualität in relevanten Bildbereichen durch Verarbeitung von Bilddaten und gezielte Rückkopplung an den Nutzer ermöglichen. Die erzielbare höchstmögliche Bildqualität kann dann eine gute Basis für einen weiteren Schritt E) zur Bewertung der Qualität eines aufgenommenen Objektes, z. B. einer Laserschnittkante, sein. Die Bewertung von Schritt E) kann dann manuell oder automatisiert erfolgen. Das beschriebene Verfahren ermöglicht es ebenfalls, z. B. ein Smartphone für die Aufnahme der Laserschnittkante zu verwenden. Das Verfahren bietet den Vorteil, dass der Nutzer unterstützt wird, ein Bild von guter Qualität von der Laserschnittkante aufzunehmen, auch wenn der Autofocus des Smartphones z. B. nicht auf die Laserschnittkante, die u. U. nur einen kleinen Teil des Bildes einnimmt, fokussiert oder wenn der Nutzer das Smartphone in der Hand nicht vollständig ruhig halten kann. Durch die Rückmeldung erhält der Nutzer die Möglichkeit, die Aufnahme zu wiederholen.

Weitere Vorteile der Erfindung ergeben sich aus der Beschreibung und der Zeichnung. Ebenso können die vorstehend genannten und die noch weiter ausgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

- Fig. 1: zeigt schematisch ein Ablaufdiagramm des Verfahrens;
- Fig. 2: zeigt schematisch ein System mit mobilem Endgerät (10) und Laserschneidmaschine (14);
- Fig. 3: zeigt schematisch mögliche Anzeigen auf dem berührungsempfindlichen Bildschirm (24);
- Fig. 4: zeigt beispielhaft Resultate von Segmentierungen;
- Fig. 5: zeigt eine schematische Darstellung einer Ausführungsform eines Systems (100) mit Laserschneidmaschine (14);
- Fig. 6: zeigt eine schematische Darstellung einer Ausführungsform eines Steuersystems (600).

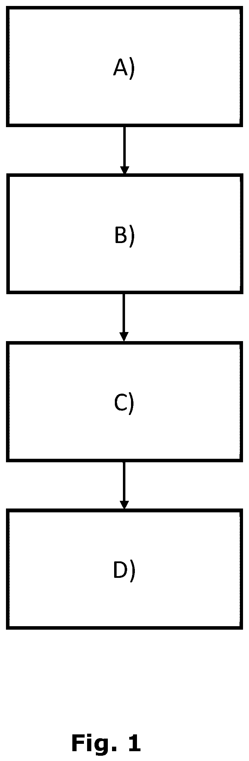

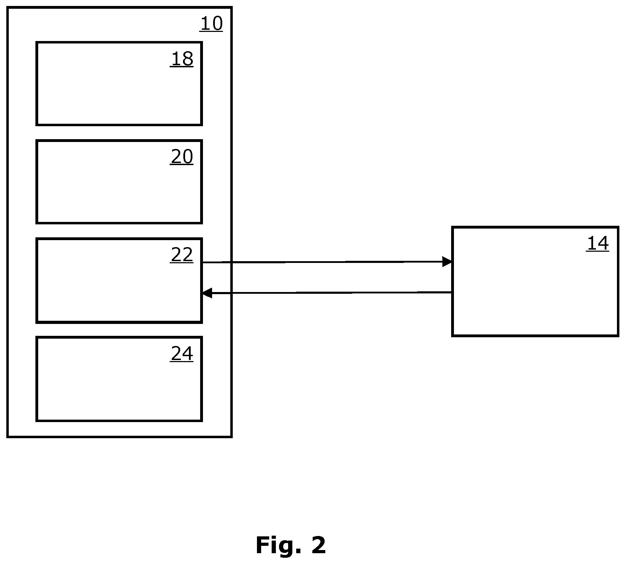

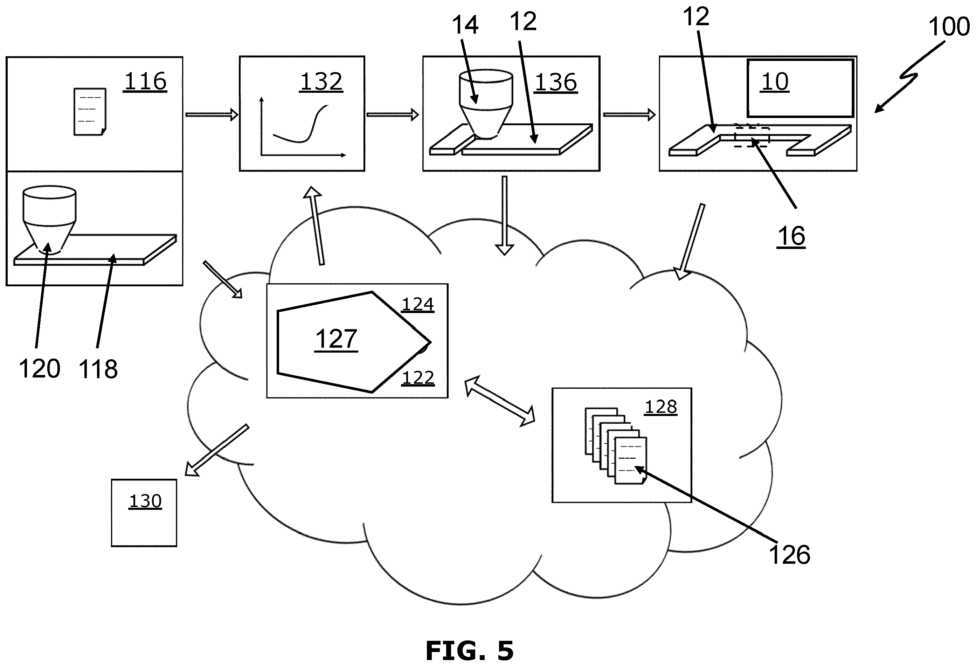

Figur 1 zeigt schematisch ein Ablaufdiagramm des Verfahrens zum Bewerten einer Laserschnittkante 16 (Fig. 5) eines Werkstückes 12 (Fig. 5) mit den Schritten A), B), C) und D). In Schritt A) werden Bilddaten der Laserschnittkante 16 und ihrer Umgebung mittels einer Kamera 18 erfasst. In Schritt B) werden die Bilddaten, insbesondere mittels eines neuronalen Netzes, segmentiert und mittels der Segmentierung ein interessierendes Segment der Bilddaten ermittelt. Für ein Verfahren zur Bewertung der Qualität einer Laserschnittkante ist die Laserschnittkante selbst der wesentliche Bereich des Bildes. Bei der Segmentierung der Bilddaten werden deshalb die Bildpunkte, Pixel, der Bilddaten dem interessierenden Segment zugeordnet, die auf der Laserschnittkante 16 liegen. Andere Bildpunkte, Pixel, werden der Umgebung der Laserschnittkante 16 zugeordnet. In Schritt C) wird dann für das interessierende Segment eine Bildqualitätserkennung, insbesondere Bildschärfeerkennung, vorzugsweise im Frequenzbereich, durchgeführt. In Schritt D) wird eine Ausgabe für einen Nutzer generiert und bevorzugt auf einem Bildschirm 24 (Fig. 2) ausgegeben. In der Ausgabe wird der Nutzer über das Ergebnis der Bildqualitätserkennung informiert und kann damit einen Hinweis erhalten, ob die Qualität des Bildes für eine evtl. nachfolgende Bewertung der Qualität der Laserschnittkante 16 in einem Schritt E) ausreichend ist oder nicht. Ist die Bildqualität nicht ausreichend für eine Bewertung der Qualität der Laserschnittkante 16, kann dem Nutzer vorgeschlagen werden, die Aufnahme des Bildes zu wiederholen. Und dies so lange, bis genügend Bilder, z. B. zumindest ein Bild, in ausreichender Qualität zur Verfügung steht/stehen.

Figur 2 zeigt schematisch ein System mit mobilem Endgerät 10 und Laserschneidmaschine 14. Das mobile Endgerät 10 weist eine Kamera 18, optional eine Tastatur 20 als Eingabeschnittstelle, eine Kommunikationsschnittstelle 22, z. B. für drahtlose Kommunikation, sowie einen berührungsempfindlichen Bildschirm 24 auf. Der Bildschirm 24 kann auch als Bildschirm ohne berührungsempfindliche Oberfläche ausgebildet sein, also keine Eingabefunktionalität aufweisen. Das mobile Endgerät ist ausgebildet, über die Kommunikationsschnittstelle 22 mit der Laserschneidmaschine 14 oder anderen Einheiten zu kommunizieren. Diese Kommunikation erfolgt bevorzugt drahtlos, es kann jedoch auch eine drahtgebundene Kommunikation vorgesehen sein. Über die Kommunikationsschnittstelle 22 kann das mobile Endgerät z. B. Parameter betreffend die Laserschnittkante 16 und damit das Werkstück 12, z. B. von der Laserschneidmaschine 14, empfangen. Diese Parameter umfassen z. B. Daten zur Art des Materials des Werkstücks 12, zur Art des Schneidverfahrens und/oder zur Dicke des Werkstücks 12.

In der Erfindung übermittelt das mobile Endgerät 10 über die Kommunikationsschnittstelle 22 ebenfalls die durch die Kamera 18 aufgenommenen Bilddaten an eine Recheneinheit und empfängt von der Recheneinheit Bilddaten. Die empfangenen Bilddaten sind segmentiert und in den empfangenen Bilddaten ist das interessierende Segment identifiziert. Die empfangenen Bilddaten weisen das Ergebnis einer Bildqualitätserkennung für das interessierende Segment auf. Die Recheneinheit wiederum ist eingerichtet, die Bilddaten zu segmentieren und ein interessierendes Segment der Bilddaten zu identifizieren, wobei das interessierende Segment Bilddaten der Laserschnittkante aufweist; und wobei die Recheneinheit eingerichtet ist, eine Bildqualitätserkennung für das interessierende Segment durchzuführen. Die erwähnte Recheneinheit kann beispielsweise in der in Figur 5 dargestellten Recheneinheit 122 realisiert sein.

Entsprechend der Erfindung ist die Recheneinheit innerhalb des mobilen Endgeräts 10 realisiert. Die Kommunikation zwischen dem mobilen Endgerät und der Recheneinheit erfolgt innerhalb des mobilen Endgeräts 10.

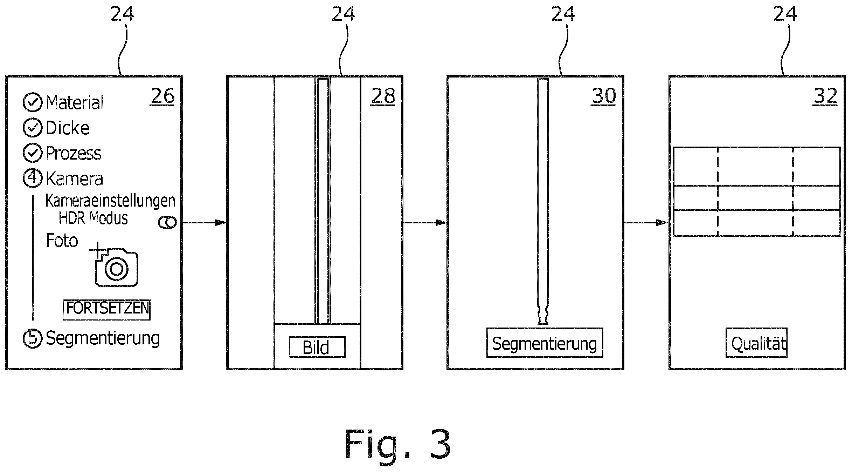

Figur 3 zeigt schematische mögliche Anzeigen, insbesondere auf dem berührempfindlichen Bildschirm 24, z. B. auf einem Mobiltelefon oder Tablet-Computer. In Bild (Anzeige) 26 wird beispielhaft die Situation vor Schritt A) des Verfahrens angezeigt, bei der Parameter zum verwendeten Material, zur verwendeten Materialdicke und zum verwendeten Prozess bereits empfangen worden sind. Dies kann beispielsweise durch eine Eingabe durch den Nutzer oder den Empfang über eine Kommunikationsschnittstelle erfolgt sein. Zugleich ist in Bild 26 erkennbar, dass die Kamera bereit zur Aufnahme ist.

In Bild (Anzeige) 28 wird beispielhaft das Resultat von Schritt A) des Verfahrens angezeigt, d. h. das von der Kamera aufgenommene Bild wird angezeigt. In Bild (Anzeige) 30 wird beispielhaft das Resultat von Schritt B) des Verfahrens angezeigt, d. h. das Ergebnis der Segmentierung wird so angezeigt, dass der Nutzer identifizieren kann, in welchem Bereich des Bildes das Verfahren die Laserschnittkante verortet.

In Bild (Anzeige) 32 wird beispielhaft das Resultat von Schritt C) und D) des Verfahrens angezeigt, d. h. das Ergebnis der Bildqualitätserkennung wird dem Nutzer ausgegeben. Im dargestellten Beispiel wird in verschiedenen Grautönen, in der Realität bevorzugt in verschiedenen Farben, angezeigt, wie die Bildschärfe in den verschiedenen Bildbereichen ist und ob sie ausreicht oder ob empfohlen wird, ein weiteres Foto aufzunehmen.

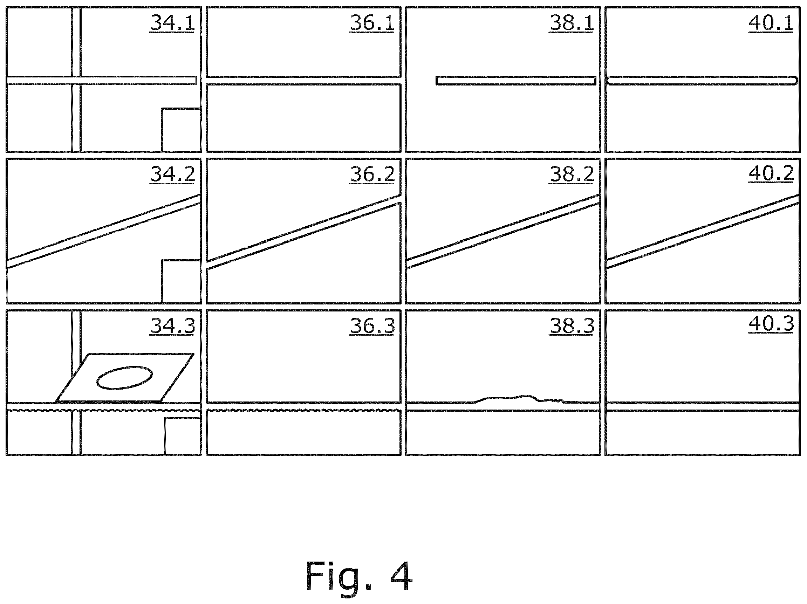

Figur 4 zeigt Beispiele von Resultaten von Segmentierungen. Die Bilder 34.1, 34.2 und 34.3 sind beispielhafte Testbilder von Laserschnittkanten. Die Bilder 36.1, 36.2, 36.3 sind jeweils zugeordnete Ground Truth Daten, d. h. die "korrekte" erwünschte Segmentierung, bei der die Schnittkante eindeutig identifiziert ist. Die Bilder 38.1, 38.2, 38.3 zeigen das Ergebnis eines ersten Segmentierungs-Algorithmus und die Bilder 40.1, 40.2, 40.3 das Ergebnis eines zweiten Segmentierungs-Algorithmus. In den Bildern 38.1, 38.2, 38.3, 40.1, 40.2, 40.3 ist zu erkennen, dass jeder der beiden Algorithmen das interessierende Segment mit der Laserschnittkante identifiziert hat - zu erkennen in dem heller gefärbten Bereich in den jeweiligen Bildern 38.1, 38.2, 38.3, 40.1, 40.2, 40.3.

Figur 5 zeigt beispielhaft ein System 100 zur Bearbeitung eines Werkstücks 12 mit einer Laserschneidmaschine 14.

Über eine Eingabeeinheit 116 wird zumindest ein Materialparameter 118, der insbesondere kennzeichnend für den verwendeten Werkstückwerkstoff und/oder dessen Dicke steht, zumindest ein Maschinenparameter 120, der insbesondere kennzeichnend für die verwendete Laserschneidmaschine 14 sowie vorzugsweise zumindest ein gewünschtes Schnittkanten-Qualitätsmerkmal eingegeben. Weiterhin kann über die Eingabeeinheit 116 zumindest ein gewünschter Verfahrensparameter, insbesondere Laserleistung, Fokustiefe, Vorschubgeschwindigkeit und/oder Gasfluss, eingegeben werden.

Das System 100 kann dazu ausgebildet sein, den verwendeten Materialparameter 118 durch messtechnische Erfassung, insbesondere durch Gewichtsmessung und Vergleich mit hinterlegten Werkstoffkenndaten, sowie die Werkstückabmaße des Werkstücks 12, insbesondere durch Vermessung des Werkstücks 12, selbständig zu bestimmen. Weiterhin kann das System 100 dazu ausgebildet sein, die verwendete Laserschneidmaschine 14 selbständig zu bestimmen. Durch solche Ausbildungen reduziert sich der Eingabeaufwand im Vorfeld einer Werkstückbearbeitung durch die Laserschneidmaschine 14.

Eine Recheneinheit 122 mit einem Verfahrensparameter-Algorithmus 124 liest die eingegebenen Materialparameter 118 und Maschinenparameter 120 sowie insbesondere das gewünschte Schnittkanten-Qualitätsmerkmal und/oder die gewünschten Verfahrensparameter ein und hinterlegt die Informationen in einem Datensatz 126 in einer Datenbank 128. Auf Grundlage der eingegebenen Informationen ermittelt der Verfahrensparameter-Algorithmus 124 die verbesserten, vorzugsweise optimalen, und/oder die zur Erreichung des gewünschten Schnittkanten-Qualitätsmerkmals erforderlichen Verfahrensparameter.

Der Verfahrensparameter-Algorithmus weist hierzu eine Daten-Aggregations-Routine 127 auf. Vorzugsweise ist der Verfahrensparameter-Algorithmus in Form der Daten-Aggregation-Routine 127 ausgebildet.

Die so ermittelten Verfahrensparameter werden über die Anzeige 130 ausgegeben und/oder an eine Steuerung 132 zur Steuerung der Laserschneidmaschine 14 weitergeleitet. Nach Bekanntgabe der verbesserten, vorzugsweise optimalen, Verfahrensparameter kann der Nutzer entweder die Verfahrensparameter-Empfehlung zur Anwendung freigeben oder eine anderweitige Einstellung der Verfahrensparameter durchführen und den Verfahrensprozess starten. Im Anschluss wird das Werkstück 12 durch die Laserschneidmaschine 14 und anhand der vorgegebenen Verfahrensparameter bearbeitet. Die für die Bearbeitung des Werkstücks 12 durch die Laserschneidmaschine 14 maßgeblichen Verfahrensparameter sowie die von dem Verfahrensparameter-Algorithmus 124 vorgeschlagenen Verfahrensparameter werden dem Datensatz 126 dieser Werkstückbearbeitung hinzugefügt.

Um eine eindeutige Zuordnung des Werkstücks 12 zum Datensatz 126 zu ermöglichen, kann eine Kennzeichnung des Werkstücks 12 innerhalb des Prozesses manuell oder automatisiert, insbesondere durch Lasergravur, vorzugsweise eines QR-Codes, insbesondere während des Laserschneidvorgangs, durchgeführt werden. Eine derartige Kennzeichnung hat zudem den Vorteil einer automatisierten Zuordnung des Werkstücks durch einfaches Scannen des Werkstücks 12 im weiteren Prozessverlauf. Wird eine entsprechende Kennzeichnung des Werkstücks 12 durchgeführt, wird dem Datensatz 126 dieser Werkstückbearbeitung eine entsprechende Information hinzugefügt.

Im Anschluss an die Bearbeitung des Werkstücks 12 wird die Qualität der entstandenen Schnittkante, insbesondere die unterschiedlichen Schnittkanten-Qualitätsmerkmale, ermittelt. Dies kann unter Verwendung des Verfahrens mit den Schritten A), B), C) und D) erfolgen. Dem Nutzer wird mittels des Schrittes D) angezeigt, ob die Qualität der optischen Aufnahme der Schnittkante 16 gut genug ist oder ob er den Aufnahmevorgang wiederholen soll. Die Bilddaten, die die Qualität der Schnittkante 16 repräsentieren, werden dem Datensatz 26 dieser Werkstückbearbeitung hinzugefügt. Vorzugsweise wird im Anschluss an die Bearbeitung des Werkstücks 12 durch die Laserschneidmaschine 14 eine objektive Bestimmung der Schnittkanten-Qualitätsmerkmale in einem Schritte E) des Verfahrens durchgeführt. Dabei wird anhand der Bilddaten die Qualität der Schnittkante 16 automatisiert mittels eines Bildverarbeitungsverfahrens in dem Schritt E) ermittelt. Vorzugsweise werden die Messergebnisse dem entsprechenden Datensatz 126 der Werkstückbearbeitung hinzugefügt.

Die Datenbank 128 ist dazu ausgebildet, alle Datensätze 26 von Werkstückbearbeitungen zu speichern. Die Datenbank 128 bildet somit die Grundlage für die Veränderung, insbesondere Verbesserung, vorzugsweise Optimierung, des Verfahrensparameter-Algorithmus 124.

Vorzugsweise werden bereits bearbeitete Werkstücke 12 hinsichtlich ihrer Schnittkanten-Qualitätsmerkmale bewertet und zur Verfahrensverbesserung hinsichtlich der Bearbeitung nachfolgender Werkstücke 12 verwendet.

Beim Laserschneiden, beispielsweise durch Sensoren, gemessene instationäre Verfahrensparameter 136 können ebenfalls in der Datenbank 128 hinterlegt werden und können den Datensatz 126 der aktuellen Werkstückbearbeitung ergänzen. Dies bietet insbesondere den Vorteil, Schwankungen in den Verfahrensparametern während des Laserschneidens zu bestimmen und in eine Bewertung der Schnittkanten-Qualität miteinzubeziehen. Dadurch kann eine besonders hohe Vorhersagefähigkeit in Bezug auf die Schnittkanten-Qualität und den Maschinenzustand erreicht werden.

Auf Basis gespeicherter Datensätze 126 in der Datenbank 128 kann eine Veränderung, insbesondere Verbesserung, vorzugsweise Optimierung, der zumindest einen, insbesondere aller, Daten-Aggregations-Routine(n) 127 des Verfahrensparameter-Algorithmus 124 erfolgen. Hierbei können auch Datensätze 126 unterschiedlicher Nutzer des Systems 100 zusammen genutzt werden, um einen verbesserten, insbesondere optimalen, Zusammenhang zwischen Eingangs- und Ausgangsparametern der zumindest einen Daten-Aggregations-Routine 127 zu bestimmen.

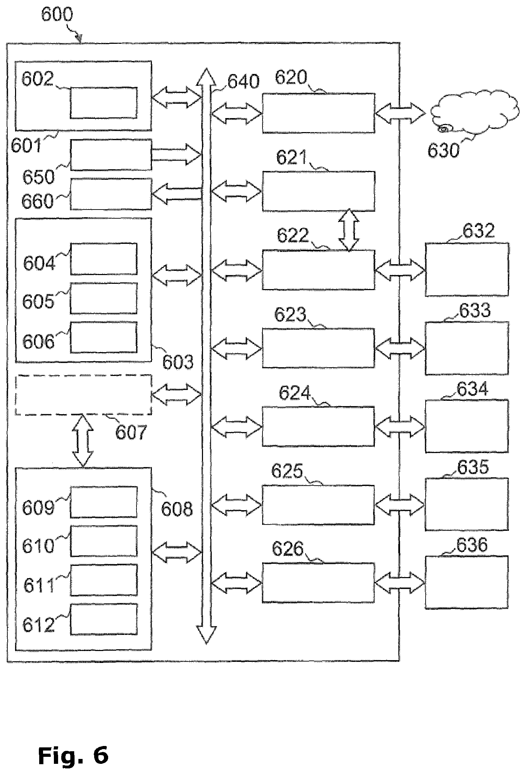

Figur 6 zeigt eine schematische Darstellung eines Steuersystems 600, das geeignet ist, Anweisungen zum Ausführen eines oder mehrerer Aspekte des Verfahrens in einer Vorrichtung auszuführen. Die Komponenten sind als Beispiele zu verstehen und beschränken nicht den Umfang der Verwendung oder Funktionalität von Hardware, Software, Firmware, eingebetteten Logikkomponenten, oder einer Kombination von mehreren solcher Komponenten zur Implementierung spezieller Ausführungsformen der vorliegenden Erfindung. Einige oder alle der dargestellten Komponenten können ein Teil des Steuersystems 600 sein.

Das Steuersystem 600 enthält in dieser Ausführungsform zumindest einen Prozessor 601, wie beispielsweise eine zentrale Verarbeitungseinheit (CPU, DSP) oder einen programmierbaren Logikbaustein (PLD, FPGA). Das Steuersystem 600 kann auch einen Arbeitsspeicher 603 und einen Datenspeicher 608 umfassen, die beide miteinander und mit anderen Komponenten über einen Bus 640 kommunizieren. Der Bus 640 kann auch eine Anzeige 632, eine oder mehrere Eingabevorrichtungen 633, eine oder mehrere Ausgabevorrichtungen 634, eine oder mehrere Speichervorrichtungen 635 und verschiedene Speichermedien 636 miteinander und mit einem oder mehreren Vorrichtungen des Prozessors 601, dem Arbeitsspeicher 603 und dem Datenspeicher 608 verbinden. Alle diese Elemente können direkt oder über eine oder mehrere Schnittstellen 622, 623, 624, 625, 626 oder Adapter mit dem Bus 640 gekoppelt werden.

Das Steuersystem 600 kann irgendeine geeignete physikalische Form haben, einschließlich, aber nicht begrenzt auf einen oder mehrere integrierte Schaltkreise (ICs), Leiterplatten (PCBs), mobile Handgeräte, Laptop-oder Notebook-Computer, verteilte Computersysteme, Rechengitter oder Server. Der Prozessor 601 oder eine zentrale Verarbeitungseinheit (CPU) enthält gegebenenfalls eine Cache-Speichereinheit 602 zum temporären lokalen Speichern von Befehlen, Daten oder Prozessoradressen. Der Prozessor 601 ist konfiguriert, um die Ausführung der Anweisungen, welche auf mindestens einem Speichermedium gespeichert sind, zu unterstützen.

Der Arbeitsspeicher 603 und der Datenspeicher 608 können jeweils als ein computerlesbares, insbesondere nichtflüchtiges, insbesondere greifbares, Speichermedium ausgestaltet sein. Sie können verschiedene Komponenten aufweisen, einschließlich, aber ist nicht zu begrenzen auf eine Direktzugriffsspeicherkomponente z. B. RAM 604 insbesondere ein statischer RAM "SRAM", ein dynamischer RAM "DRAM, usw., eine Nur-Lese-Komponente, z. B. ROM 605, und beliebige Kombinationen davon. Der ROM 605 kann auch fungieren, um Daten und Anweisungen unidirektional an den oder die Prozessoren 601 zu kommunizieren, und der RAM 604 kann auch fungieren, um Daten und Anweisungen bidirektional an den oder die Prozessoren 601 zu kommunizieren.

Die Speicher 603, 608 und Speichermedien können bidirektional mit dem oder den Prozessoren 601, wahlweise durch eine Speichersteuereinheit 607, verbunden sein. Beide Speicher 608, 603 können verwendet werden, um das Betriebssystem 609, Programme 610, Daten 611, Anwendungen 612, Anwendungsprogramme, und dergleichen zu speichern. Häufig, jedoch nicht immer, sind die Speicher 603, 608 durch ein sekundäres Speichermedium (wie eine Festplatte), das langsamer als der Primärspeicher (z. B. Speicher 603) ist, unterstützt. Der Speicher 603, 608 kann z.B. auch eine magnetische, eine optische oder eine transistorisierte, eine Festkörper-Speichervorrichtung (z. B. Flash-basierte Systeme) oder eine Kombination von beliebigen der oben genannten Elemente umfassen.

Der Bus 640 verbindet eine Vielzahl von Subsystemen. Der Bus 640 kann ein beliebiger von mehreren Typen von Busstrukturen sein, z.B. ein Speicherbus, ein Speichercontroller, ein peripherer Bus, ein lokaler Bus, und alle Kombinationen davon, unter Verwendung einer Vielzahl von Busarchitekturen. Informationen und Daten können auch über eine Anzeige 632 angezeigt werden. Beispiele für eine Anzeige 632 umfassen, sind aber nicht beschränkt auf, eine Flüssigkristallanzeige (LCD), ein organisches Flüssigkristall-Display (OLED), eine Kathodenstrahlröhre (CRT), eine Plasmaanzeige, und beliebige Kombinationen davon. Die Anzeige 632 kann mit Prozessor(en) 601, Speichern 603, 608, Eingabegeräten 633, und weiteren Komponenten über den Bus 640 verbunden sein.

Der Bus 640 kann alle zuvor genannten Komponenten mit einer Netzwerkschnittstelle 620 mit einem externen Netzwerk 630 verbinden. Das kann z.B. ein LAN, WLAN, etc. sein. Es kann eine Verbindung zu weiteren Speichermedien, Servern, Druckern, Anzeigegeräten aufbauen. Es kann einen Zugang zum Telekommunikationsvorrichtungen und Internet aufweisen. Der Bus 640 kann alle zuvor genannten Komponenten mit einer Grafiksteuerung 621 und einer Grafikschnittstelle 622 verbinden, die mit zumindest einer Eingabevorrichtungen 633 verbindbar ist.

Der Bus 640 kann alle zuvor genannten Komponenten mit einer Eingabeschnittstelle 623 verbinden, die mit zumindest einer Eingabevorrichtung 633 verbindbar ist. Eine Eingabevorrichtung kann z.B. ein Tastenfeld, eine Tastatur, eine Maus, ein Stift, ein Touchscreen usw. einschließen.

Der Bus 640 kann alle zuvor genannten Komponenten mit einer Ausgabeschnittstelle 624 verbinden, die mit zumindest einer Ausgabevorrichtung 634 verbindbar ist. Eine Ausgabevorrichtungen 634 kann eine Leuchtanzeige, eine LED Anzeige ein Display, z.B. LCD, OLED usw. oder eine Schnittstelle zu einer solchen Einrichtung aufweisen.

Der Bus 640 kann alle zuvor genannten Komponenten mit einer Speicherzugriffsschnittstelle 625 verbinden, die mit zumindest einer Speichervorrichtung 635 verbindbar ist. Der Bus 640 kann alle zuvor genannten Komponenten mit einer weiteren Speicherzugriffsschnittstelle 626 verbinden, die mit zumindest einem Speichermedium 636 verbindbar ist. Eine Speichervorrichtung 635 oder ein Speichermedium 636 kann z.B. ein Festkörper-, ein Magnetspeicher oder ein optischer Speicher sein, insbesondere einen nichtflüchtigen Speicher aufweisen. Das Speichermedium kann im Betrieb des Steuersystems vom Steuersystem getrennt werden, ohne dass Daten verloren gehen. Das Speichermedium kann greifbar sein, also ein materiell vorhandener Gegenstand sein.

Der Bus 640 kann ganz oder teilweise durch Kabel oder Leitungen (z.B. LAN, RS232 etc.) oder ganz oder teilweise kabellos per Funkverbindung oder dergleichen (z.B. WLAN, WIFI, Buetooth, NFC etc.) realisiert sein.

Anzeige 632, Eingabevorrichtung 633, Ausgabevorrichtung 634, Speichervorrichtung 635 und/oder Speichermedium 636 können jeweils außerhalb des Steuersystems 600 angeordnet sein oder in ihm integriert sein. Sie können auch über eine Verbindung zum Internet oder anderen Netzwerkschnittstellen mit dem Steuersystem 600 verbunden sein.

## Patentansprüche

1. Verfahren zum Bewerten einer Laserschnittkante (16) eines Werkstücks (12), aufweisend die Schritte
A) Erfassen von Bilddaten der Laserschnittkante (16) und ihrer Umgebung;
B) Segmentierung der Bilddaten und Identifikation eines interessierenden Segmentes der Bilddaten;
C) Durchführung einer Bildqualitätserkennung für das interessierende Segment;
D) Generierung einer Ausgabe für einen Nutzer, basierend auf der Bildqualitätserkennung, wobei der Nutzer in der Ausgabe über das Ergebnis der Bildqualitätserkennung informiert wird,
**dadurch gekennzeichnet, dass** das interessierende Segment Bilddaten der Laserschnittkante (16) aufweist, wobei in Schritt B) für jedes Pixel der Bilddaten ermittelt und abgespeichert wird, ob es einen Teil der Laserschnittkante (16) repräsentiert und bei der Segmentierung der Bilddaten die Bildpunkte, Pixel, der Bilddaten dem interessierenden Segment zugeordnet werden, die auf der Laserschnittkante (16) liegen.

2. Verfahren nach Anspruch 1, **dadurch gekennzeichnet, dass** Schritt B) mittels eines neuronalen Netzes ausgeführt wird.

3. Verfahren nach einem der vorhergehenden Ansprüche, **dadurch gekennzeichnet, dass** die Bildqualitätserkennung eine Schärfeerkennung umfasst.

4. Verfahren nach Anspruch 3, **dadurch gekennzeichnet, dass** die Schärfeerkennung eine Transformation der Bilddaten des interessierenden Segments in den Frequenzbereich umfasst und dass die Schärfeerkennung im Frequenzbereich durchgeführt wird.

5. Verfahren nach einem der vorhergehenden Ansprüche, **dadurch gekennzeichnet, dass** vor Schritt A) die Laserschnittkante (16) mittels eines Laserstrahls und insbesondere zusätzlich mittels eines Druckluftstrahls, geschnitten wird.

6. Verfahren nach einem der vorhergehenden Ansprüche, **dadurch gekennzeichnet, dass** vor Schritt A) Parameter betreffend die Laserschnittkante (16) empfangen werden, wobei die Parameter insbesondere Daten zur Art des Materials des Werkstücks 12, zur Art des Schneidverfahrens und/oder zur Dicke des Werkstücks 12 sind.

7. Mobiles Endgerät (10), aufweisend
• eine Kamera (18), die eingerichtet ist, Bilddaten einer Laserschnittkante (16) mit Umgebung zu erfassen;
**dadurch gekennzeichnet, dass**:
das mobile Endgerät (10) eingerichtet ist,
• die Bilddaten an eine Recheneinheit zu übermitteln und von der Recheneinheit Bilddaten zu empfangen, wobei die empfangenen Bilddaten segmentiert sind und in den empfangenen Bilddaten ein interessierendes Segment identifiziert ist, wobei das interessierende Segment Bilddaten der Laserschnittkante (16) aufweist; und wobei die empfangenen Bilddaten das Ergebnis einer Bildqualitätserkennung für das interessierende Segment aufweisen,
wobei das mobile Endgerät die Recheneinheit aufweist,
wobei die Recheneinheit eingerichtet ist, die Bilddaten zu segmentieren und ein interessierendes Segment der Bilddaten zu identifizieren und wobei die Recheneinheit eingerichtet ist, eine Bildqualitätserkennung für das interessierende Segment durchzuführen,
wobei das mobile Endgerät (10) eine Ausgabeschnittstelle (24) aufweist, die eingerichtet ist, eine Ausgabe für einen Nutzer zu generieren, wobei die Ausgabe auf dem Ergebnis der Bildqualitätserkennung basiert, wobei die Ausgabe über das Ergebnis der Bildqualitätserkennung informiert und, wobei das interessierende Segment Bilddaten der Laserschnittkante (16) aufweist, wobei die Recheneinheit eingerichtet ist für jedes Pixel der Bilddaten zu ermitteln und abzuspeichern, ob es einen Teil der Laserschnittkante (16) repräsentiert und bei der Segmentierung der Bilddaten die Bildpunkte, Pixel, der Bilddaten dem interessierenden Segment zuzuordnen, die auf der Laserschnittkante (16) liegen.

8. Mobiles Endgerät nach Anspruch 7, **dadurch gekennzeichnet, dass** das mobile Endgerät (10) als Mobiltelefon, Tablet-Computer oder Webcam ausgebildet ist.

9. Mobiles Endgerät nach einem der Ansprüche 7 bis 8, **gekennzeichnet durch** mindestens eine Schnittstelle (20, 22, 24) zum Empfang von Parametern betreffend die Laserschnittkante (16).

10. Mobiles Endgerät nach Anspruch 9, **dadurch gekennzeichnet, dass** die mindestens eine Schnittstelle als Eingabeschnittstelle (20, 22) für einen Nutzer und/oder als Kommunikationsschnittstelle (24) zur Kommunikation mit einer Laserschneidmaschine (14) ausgebildet ist.

11. System umfassend
• eine Laserschneidmaschine (14), die eingerichtet ist, mittels eines Laserstrahls eine Laserschnittkante (16) in ein Werkstück (12) zu schneiden und
**gekennzeichnet durch**:
• ein mobiles Endgerät (10) nach einem der Ansprüche 7 bis 10.

## Claims

1. A method for evaluating a laser cutting edge (16) of a workpiece (12), comprising the steps of
A) capturing image data of the laser cutting edge (16) and the surroundings thereof;
B) segmenting the image data and identifying a segment of interest of the image data;
C) carrying out an image quality detection for the segment of interest;
D) generating an output for a user based on the image quality detection, wherein the user is informed of the result of the image quality detection in the output,
**characterised in that** the segment of interest comprises image data of the laser cutting edge (16), wherein in step B) for each pixel of the image data it is determined and stored whether it represents a part of the laser cutting edge (16) and, during the segmentation of the image data, the image points, pixels, of the image data that lie on the laser cutting edge (16) are assigned to the segment of interest.

2. The method according to claim 1, **characterised in that** step B) is carried out using a neural network.

3. The method according to either one of the preceding claims, **characterised in that** the image quality detection comprises a sharpness detection.

4. The method according to claim 3, **characterised in that** the sharpness detection comprises a transformation of the image data of the segment of interest into the frequency domain, and **in that** the sharpness detection is carried out in the frequency domain.

5. The method according to any one of the preceding claims, **characterised in that** before step A) the laser cutting edge (16) is cut by means of a laser beam and in particular additionally by means of a compressed air jet.

6. The method according to any one of the preceding claims, **characterised in that** parameters relating to the laser cutting edge (16) are received before step A), wherein the parameters are in particular data on the type of material of the workpiece 12, on the type of cutting method and/or on the thickness of the workpiece 12.

7. A mobile terminal (10) comprising
• a camera (18) configured to capture image data of a laser cutting edge (16) and the surroundings thereof;
**characterised in that**:
the mobile terminal (10) is configured
• to transmit the image data to a computing unit and to receive image data from the computing unit, wherein the received image data are segmented and a segment of interest is identified in the received image data, wherein the segment of interest comprises image data of the laser cutting edge (16); and wherein the received image data comprise the result of an image quality detection for the segment of interest,
wherein the mobile terminal comprises the computing unit, wherein the computing unit is configured to segment the image data and to identify a segment of interest of the image data, and wherein the computing unit is configured to perform an image quality detection for the segment of interest,
wherein the mobile terminal (10) has an output interface (24) configured to generate an output for a user, wherein the output is based on the result of the image quality detection, wherein the output provides information about the result of the image quality detection, and wherein the segment of interest comprises image data of the laser cutting edge (16), wherein the computing unit is configured to determine and to store for each pixel of the image data whether it represents a part of the laser cutting edge (16), and during the segmentation of the image data to assign to the segment of interest the image points, pixels, of the image data that lie on the laser cutting edge (16).

8. The mobile terminal according to claim 7, **characterised in that** the mobile terminal (10) is designed as a mobile phone, tablet computer or webcam.

9. The mobile terminal according to any one of claims 7 to 8, **characterised by** at least one interface (20, 22, 24) for receiving parameters relating to the laser cutting edge (16).

10. The mobile terminal according to claim 9, **characterised in that** the at least one interface is designed as an input interface (20, 22) for a user and/or as a communication interface (24) for communication with a laser cutting machine (14).

11. A system comprising
• a laser cutting machine (14) configured to cut a laser cutting edge (16) into a workpiece (12) using a laser beam and
**characterised by**:
• a mobile terminal (10) according to any one of claims 7 to 10.

## Revendications

1. Procédé d'évaluation d'une arête de coupe au laser (16) d'une pièce (12), comprenant les étapes suivantes :
A) l'acquisition des données d'image de l'arête de coupe au laser (16) et de son environnement ;
B) la segmentation des données d'image et l'identification d'un segment d'intérêt dans les données d'image ;
C) la réalisation d'une détection de la qualité d'image pour le segment d'intérêt ;
D) la génération d'une sortie pour un utilisateur, basée sur la détection de la qualité de l'image, dans lequel l'utilisateur est informé du résultat de la détection de la qualité de l'image,
**caractérisé en ce que** le segment d'intérêt comprend les données d'image de l'arête de coupe au laser (16), dans lequel l'étape B) consiste à déterminer et à mémoriser, pour chaque pixel des données d'image, s'il représente une partie de l'arête de coupe laser (16), et lors de la segmentation des données d'image, des points d'image, à savoir les pixels des données d'image, lesquels sont situés sur l'arête de coupe au laser (16), sont attribués au segment d'intérêt.

2. Procédé selon la revendication 1, **caractérisé en ce que** l'étape B) est réalisée à l'aide d'un réseau neuronal.

3. Procédé selon l'une quelconque des revendications précédentes, **caractérisé en ce que** la détection de la qualité d'image comprend la détection de la netteté.

4. Procédé selon la revendication 3, **caractérisé en ce que** la détection de netteté comprend une transformation des données d'image du segment d'intérêt dans le domaine fréquentiel et que la détection de netteté est réalisée dans le domaine fréquentiel.

5. Procédé selon l'une quelconque des revendications précédentes, **caractérisé en ce que**, avant l'étape A), l'arête de coupe au laser (16) est coupée au moyen d'un faisceau laser et, en particulier en outre au moyen d'un jet d'air comprimé.

6. Procédé selon l'une quelconque des revendications précédentes, **caractérisé en ce que** des paramètres relatifs à l'arête de coupe au laser (16) sont reçus avant l'étape A), dans lequel les paramètres sont, en particulier des données sur le type de matière de la pièce 12, sur le type de procédé de coupe et/ou sur l'épaisseur de la pièce 12.

7. Terminal (10) mobile, comportant :
• une caméra (18), laquelle est conçue pour capturer des données d'image d'une arête de coupe au laser (16) et de son environnement ;
**caractérisé en ce que** :
le terminal (10) mobile est conçu
• pour transmettre les données d'image à une unité de calcul et pour recevoir des données d'image de ladite unité, dans lequel les données d'image reçues sont segmentées et un segment d'intérêt est identifié dans les données reçues, dans lequel le segment d'intérêt comprend les données d'image de l'arête de coupe au laser (16) ; et dans lequel les données d'image reçues comprennent le résultat d'une détection de la qualité d'image pour le segment d'intérêt,
dans lequel le terminal mobile comprend l'unité de calcul, dans lequel l'unité de calcul est configurée pour segmenter les données d'image et pour identifier le segment d'intérêt dans les données d'image, et dans lequel l'unité de calcul est configurée pour réaliser la détection de la qualité d'image pour le segment d'intérêt,
dans lequel le terminal (10) mobile comprend une interface de sortie (24), laquelle est conçe pour générer une sortie pour un utilisateur, dans lequel la sortie est basée sur le résultat de la détection de la qualité d'image, dans lequel la sortie informe du résultat de la détection de la qualité d'image, dans lequel le segment d'intérêt comprend les données d'image de l'arête de coupe au laser (16), dans lequel l'unité de calcul est configurée pour déterminer et mémoriser, pour chaque pixel des données d'image, s'il représente une partie de l'arête de coupe au laser (16), et pour attribuer, lors de la segmentation des données d'image, au segment d'intérêt des points d'image, à savoir les pixels des données d'image, lesquels sont situés sur l'arête de coupe au laser (16).

8. Terminal mobile selon la revendication 7, **caractérisé en ce que** le terminal (10) mobile est conçu sous la forme d'un téléphone mobile, d'un ordinateur-tablette ou d'une webcam.

9. Terminal mobile selon l'une quelconque des revendications 7 à 8, **caractérisé par** au moins une interface (20, 22, 24) pour la réception des paramètres relatifs à l'arête de coupe au laser (16).

10. Terminal mobile selon la revendication 9, **caractérisé en ce que** l'au moins une interface est conçue sous la forme d'une interface d'entrée (20, 22) pour un utilisateur et/ou sous la forme d'une interface de communication (24) pour la communication avec une machine à couper au laser (14).

11. Système comprenant :
• une machine à couper au laser (14) conçue pour couper une arête de coupe au laser (16) dans une pièce (12) à l'aide d'un faisceau laser et
**caractérisé par** :
• un terminal (10) mobile selon l'une quelconque des revendications 7 à 10.
